# EUROPEAN PATENT APPLICATION

(11) **EP 1 097 671 A2**
(43) Date of publication of application: **09.05.2001**
(21) Application number: 00309592.4
(22) Date of filing: 31.10.2000
(51) Int. Cl.: A61B 5/00

(54) **Remote medical service system using communications network and method therefor**

(30) Priority: 05.11.1999 KR 9948888
(71) Applicant: SAMSUNG ELECTRONICS CO., LTD., Suwon-City, Kyungki-do (KR)
(72) Inventor: Lee, Eun-chul, Kangnam-gu, Seoul (KR)
(74) Representative: Chugg, David John

(57) **Abstract**

A remote medical service system and a method therefor, and more particularly, a remote medical service system using a communications network and a method therefor, by which the condition of a patient is determined at any time by adding portable medical equipment (103) to a communications terminal (102), and the patient receives remote medical service by connecting to a medical institution (101) through the communications network when it is determined that the patient is in a serious condition, are provided. According to the remote medical service system and the method therefor, it is possible to immediately give first aid to a patient when the patient is in serious condition, and to give a remote medical service, while checking information on the condition of the patient at any time. This is carried out by determining the condition of the patient at any time through portable medical equipment (103), and when the patient is in a condition more serious than a set basic condition of the patient, by immediately connecting to the medical institution through the communications network, transmitting the information on the condition of the patient, writing a prescription according to the remote medical service on the basis of the transmitted information, and by automatically giving the remote medical service at regular intervals.

## Description

The present invention relates to a remote medical service system and a method therefor, and more particularly, to a remote medical service system using a communications network and a method therefor.

In a conventional remote medical service method, a patient makes an appointment with a doctor, the condition of the patient is perceptually determined through a visual picture or through conversation, which is obtained by a video conference between the doctor and the patient using a communications system enabling visual communication at a determined time, and the doctor advises or prescribes medicine for the patient.

According to the conventional method, it is difficult to immediately take measures through a remote medical service when the patient is in serious condition and it is difficult to correctly examine the condition of the patient since the medical institution cannot know data required for examining the patient such as the blood pressure and pulse frequency of the patient during the remote medical service.

It is an aim of embodiments of the present invention to provide a remote medical service system using a communications network and a method therefor by which the condition of a patient is determined at any time through portable medical equipment, information on the condition of the patient is immediately transmitted to a medical institution by connecting through the communications network when the patient is in a condition more serious than the normal condition of the patient, a prescription according to the remote medical service is written on the basis of the transmitted condition, and the remote medical service is automatically given at regular intervals.

According to a first aspect of the present invention, there is provided a remote medical service system, comprising: portable medical equipment for determining the condition of a patient at predetermined time intervals, encoding the condition of the patient, and transmitting the encoded condition of the patient; and a patient terminal for operating a remote medical service program for comparing information on the condition of a patient received from the portable medical equipment with initially set basic patient condition determining information, and when it is determined that the patient is in abnormal condition, for automatically connecting to a terminal of a pre-determined medical institution and giving remote medical service.

Preferably, the patient terminal operates the remote medical service program at pre-set times according to a remotely set medical service schedule in cases other than the case where the patient is in abnormal condition.

Preferably, the remote medical service program executes a process of updating the basic patient condition determining information stored in the patient terminal (102) through a communications network according to the change in the condition of the patient in cases other than the case where the patient is in abnormal condition.

Preferably, the remote medical service program executes processes of connecting to the site address of the medical institution through a communications network, transmitting data on the condition of the patient to the terminal of the medical institution, and giving medical service through a video conference.

Preferably, the terminal of the medical institution updates the basic patient condition determining information stored in the patient terminal through the communications network according to the change in the condition of the patient as determined by the remote medical service program.

Preferably, the remote medical service system executes processes of connecting to the address of an emergency medical institution stored in the data base of the patient terminal and automatically transmitting a message announcing that the patient is in serious condition to the emergency medical institution when the patient is in serious condition.

According to a second aspect of the invention, there is provided a method for giving remote medical service using a communications system including a terminal having portable medical equipment and a data base, comprising the steps of: (a) setting information on the condition of a patient, basic patient condition determining information, information on medical institutions, and remote medical service schedules and storing them in the data base; (b) comparing information on the condition of the patient input through the portable medical equipment with the initially set basic patient condition determining information stored in the data base when a predetermined condition is met; and (c) operating a remote medical service program for automatically connecting to a terminal of an initially set medical institution through a communications network and giving remote medical service when it is determined in the step (b) that the patient is in abnormal condition.

Preferably, the remote medical service program executes processes of connecting to the site address of the set medical institution set through the communications network, transmitting the information on the condition of the patient to the terminal of the medical institution, and giving medical service through a video conference.

Preferably, the predetermined condition is whether or not a patient condition determining time included in the remote medical service schedule has arrived.

The method may further comprise the step of connecting to the address in the emergency medical institution for first aid, the address stored in the data base, and automatically transmitting a message that the patient is in serious condition when it is determined in the step (b) that the patient is in serious condition.

The method may further comprise the step of automatically operating a remote medical service program every remote medical service time included in the remote medical service schedule and giving remote medical service through the communications network when it is determined in the step (b) that the patient is in normal condition.

Preferably, the remote medical service program further executes a process of updating the basic patient condition determining information stored in the data base by data received through the terminal of the medical institution even when it is determined in the step (b) that the patient is in normal condition.

The remote medical service program may further execute a process of updating the initially set basic patient condition determining information stored in the data base with the data received through the terminal of the medical institution after giving the medical service to the patient.

For a better understanding of the invention, and to show how embodiments of the same may be carried into effect, reference will now be made, by way of example, to the accompanying diagrammatic drawings in which:
Figure 1 is a block diagram showing a remote medical service communications system to which the present invention is applied;
Figure 2 is a detailed block diagram showing the patient terminal and the portable medical equipment shown in Figure 1; and
Figure 3 is a flowchart showing a remote medical service method using a communications network according to an embodiment of the present invention.

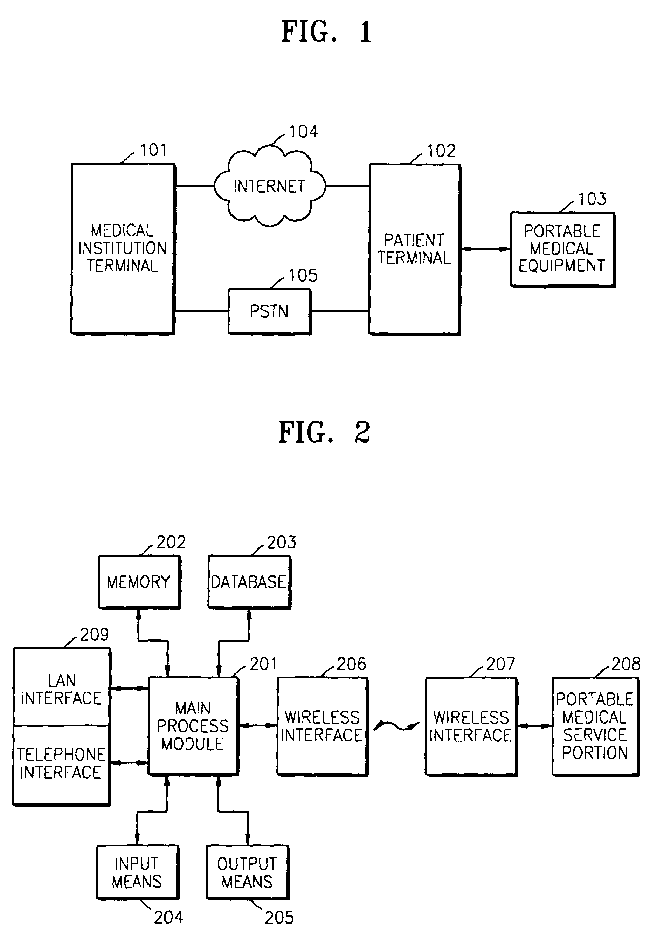

As shown in Figure 1, a remote medical service communications system to which the present invention is applied includes a medical institution terminal 101, a patient terminal 102, portable medical equipment 103, an Internet 104, and a public switched telephone network (PSTN) 105.

Figure 2 is a detailed block diagram showing the structures of the patient terminal 102 and the portable medical equipment 103 shown in Figure 1. The patient terminal 102 and the portable medical equipment 103 include a main process module 201, a memory 202, a data base 203, an input means 204, an output means 205, wireless interfaces 206 and 207, a portable medical service portion 208, and a LAN/telephone interface 209.

Patient condition determining information such as the blood pressure, the pulse frequency and the electrocardiogram of the patient, and the range of data for determining the abnormal and serious conditions of the patient, information on medical institutions, and information on remote medical service schedules on which arrangements were made with the medical institutions are stored in the data base 203.

Common executable programs for operating an image terminal and various remote medical service programs for executing remote medical service are stored in the memory 202.

The main process module 201 processes picture and audio data and controls peripheral blocks so that remote medical service can be given.

The wireless interfaces 206 and 207 transmit and receive data over radio waves.

The LAN/telephone interface 209 performs data transmission/reception interfacing for transmitting and receiving data through a LAN cable or a telephone line during connecting to the terminal of a medical institution.

A user inputs the condition of a patient or an executable instruction to a terminal using the input means 204 which may be a key board.

A prescription received from the terminal of the medical institution is output using the output means 205 which may be a monitor or a printer.

The portable medial service portion 208 checks the condition of a patient with respect to items such as the blood pressure, the pulse frequency and the electrocardiogram of the patient and outputs the measurement data.

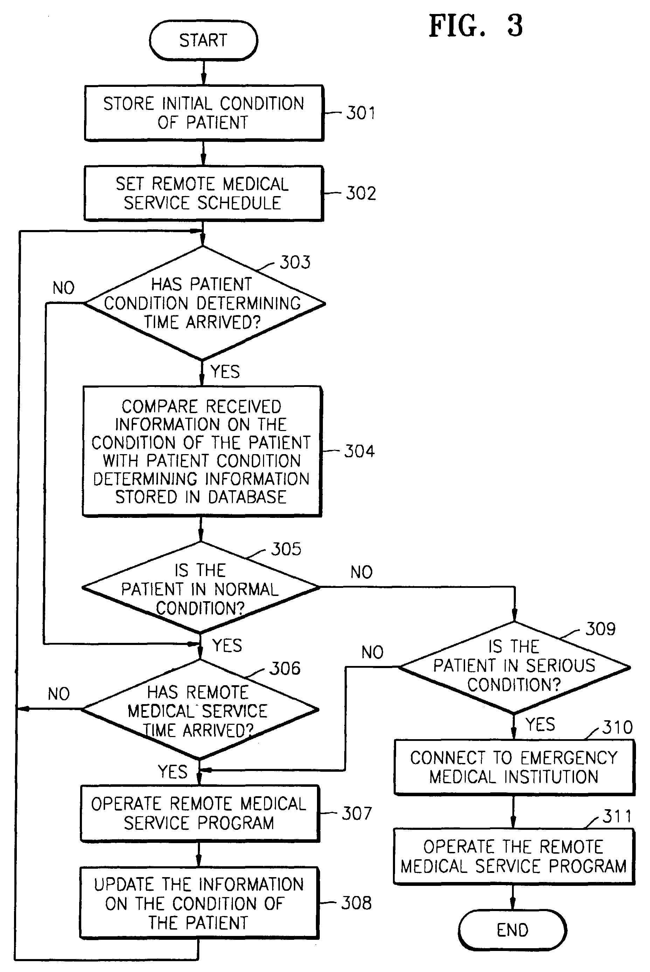

The remote medical service method using the communications network having the structure shown in Figure 2 will now be described with reference to the flowchart of Figure 3.

Patient condition determining information is checked item by item and a range of data for determining abnormal and serious conditions of the patient, information on medical institutions, and information on remote medical service schedules on which arrangements were made with the medical institutions are initially set and stored in the data base 203 (steps 301 and 302).

The time of a timer loaded into the main process module 201 is checked and it is determined whether or not a time for the condition of a patient included in the information on remote medical service schedules to be determined has arrived (step 303). When the time of the timer has arrived at the time when the condition of the patient is determined, information that the time where the condition of the patient is determined has arrived is announced to the patient through the output means 205, the information on the condition of the patient checked by the portable medical service portion 208 of the portable medical equipment 103 attached to the patient is received by the main process module 201 through the wireless interfaces 206 and 207, the received information on the condition of the patient is compared with the patient condition determining information stored in the data base 203, and it is determined whether the patient is in an abnormal condition (steps 304 and 305).

When it is determined in the step 305 that the patient is in an abnormal condition, it is determined whether the patient is in a serious condition (step 309). When it is determined that the patient is in a serious condition, the main process module 201 reads information on the Internet connection address or the telephone number of an emergency medical institution stored in the data base 203, connects to the emergency medical institution through the communications network by operating an automatic Internet connection program or a phone-calling program stored in the memory 202, and transmits a message announcing that the patient is in serious condition to the emergency medical institution through the Internet 104 or the PSTN 105 (step 310) for first aid.

The main process module 201 operates the remote medical service program stored in the memory 202, transmits the information on the condition of the patient checked by the terminal of the emergency medical institution through the communications network, and enables an emergency remote medical service to be given through a video conference (step 311).

However, when it is determined in the step 305 that the checked condition of the patient is normal, it is determined whether an initially set remote medical service schedule time has arrived (step 306). When it is determined in the step 306 that the remote medical service time has arrived or when it is determined in the step 309 that the patient is in an abnormal but not serious condition, connection to the address of a set medical institution is performed through the communications network by operating the remote medical service program, the checked information on the condition of the patient is transmitted to the medical institution terminal 101, and remote medical service is given through video conference (step 307).

After the remote medical service is completed, the information on the condition of the patient stored in the data base 203 is updated by the data received from the medical institution terminal 101 according to the change in the condition of the patient (step 308).

In an embodiment of the present invention, the data of the patient terminal 102 and the portable medical equipment 103 are transmitted over radio waves. However, it is apparent that data can be transmitted by wire communications.

As described above, according to the present invention, it is possible to immediately give first aid to a patient when the patient is in serious condition and to give remote medical service, while checking information on the condition of the patient, at any time. This is carried out by determining the condition of the patient at any time through portable medical equipment, and when the patient is in a condition more serious than a set basic condition of the patient, by immediately connecting to a medical institution through a communications network, transmitting the information on the condition of the patient, writing a prescription according to the remote medical service on the basis of the transmitted information, and automatically giving the remote medical service at regular intervals.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extend to any novel one, or any novel combination, of the features disclosed in this specification (including any accompanying claims, abstract and drawings), or to any novel one, or any novel combination, of the steps of any method or process so disclosed.

## Claims

1. A remote medical service system, comprising:
portable medical equipment (103) for determining the condition of a patient at predetermined time intervals, encoding the condition of the patient, and transmitting the encoded condition of the patient; and
a patient terminal (102) for operating a remote medical service program for comparing information on the condition of a patient received from the portable medical equipment (103) with initially set basic patient condition determining information, and when it is determined that the patient is in abnormal condition, for automatically connecting to a terminal (101)of a pre-determined medical institution and giving remote medical service.

2. The remote medical service system of claim 1, wherein the patient terminal (102) operates the remote medical service program at pre-set times according to a remotely set medical service schedule in cases other than the case where the patient is in abnormal condition.

3. The remote medical service system of claim 1 or 2, wherein the remote medical service program executes a process of updating the basic patient condition determining information stored in the patient terminal (102) through a communications network according to the change in the condition of the patient in cases other than the case where the patient is in abnormal condition.

4. The remote medical service system of claim 1, 2 or 3, wherein the remote medical service program executes processes of connecting to the site address of the medical institution through a communications network, transmitting data on the condition of the patient to the terminal (101) of the medical institution, and giving medical service through a video conference.

5. The remote medical service system of any preceding claim, wherein the terminal (101) of the medical institution updates the basic patient condition determining information stored in the patient terminal (102) through the communications network according to the change in the condition of the patient as determined by the remote medical service program.

6. The remote medical service system of any preceding claim, wherein the remote medical service system executes processes of connecting to the address of an emergency medical institution stored in the data base of the patient terminal (102) and automatically transmitting a message announcing that the patient is in serious condition to the emergency medical institution when the patient is in serious condition.

7. A method for giving remote medical service using a communications system including a terminal (102) having portable medical equipment (103) and a data base, comprising the steps of:
(a) setting information on the condition of a patient, basic patient condition determining information, information on medical institutions, and remote medical service schedules and storing them in the data base;
(b) comparing information on the condition of the patient input through the portable medical equipment (103) with the initially set basic patient condition determining information stored in the data base when a predetermined condition is met; and
(c) operating a remote medical service program for automatically connecting to a terminal (101) of an initially set medical institution through a communications network and giving remote medical service when it is determined in the step (b) that the patient is in abnormal condition.

8. The method of claim 7, wherein the remote medical service program executes processes of connecting to the site address of the set medical institution set through the communications network, transmitting the information on the condition of the patient to the terminal (101) of the medical institution, and giving medical service through a video conference.

9. The method of claim 7 or 8, wherein the predetermined condition is whether or not a patient condition determining time included in the remote medical service schedule has arrived.

10. The method of claim 7, 8 or 9, further comprising the step of connecting to the address in the emergency medical institution for first aid, the address stored in the data base, and automatically transmitting a message that the patient is in serious condition when it is determined in the step (b) that the patient is in serious condition.

11. The method of claim 7, 8, 9 or 10, further comprising the step of automatically operating a remote medical service program every remote medical service time included in the remote medical service schedule and giving remote medical service through the communications network when it is determined in the step (b) that the patient is in normal condition.

12. The method of claim 11, wherein the remote medical service program further executes a process of updating the basic patient condition determining information stored in the data base by data received through the terminal of the medical institution even when it is determined in the step (b) that the patient is in normal condition.

13. The method of any of claims 7 to 12, wherein the remote medical service program further executes a process of updating the initially set basic patient condition determining information stored in the data base with the data received through the terminal of the medical institution after giving the medical service to the patient.
